# FASCICULE DE BREVET EUROPEEN

(11) **EP 2 417 443 B1**
(45) Date de publication et mention de la délivrance du brevet: **19.06.2019**
(21) Numéro de dépôt: 10713201.1
(22) Date de dépôt: 08.04.2010
(51) Int. Cl.: G01N 27/90

(54) **DISPOSITIF DE CONTROLE NON DESTRUCTIF D'UNE STRUCTURE ELECTRIQUEMENT CONDUCTRICE**
VORRICHTUNG ZUR ZERSTÖRUNGSFREIEN ÜBERPRÜFUNG EINER ELEKTRISCH LEITENDEN STRUKTUR
DEVICE FOR NON DESTRUCTIVE TESTING OF AN ELECTRICALLY CONDUCTING STRUCTURE

(30) Priorité: 10.04.2009 FR 0952403
(43) Date de publication de la demande: 15.02.2012
(73) Titulaire: Commissariat à l'Énergie Atomique et aux Énergies Alternatives, 75015 Paris (FR)
(72) Inventeur: DECITRE, Jean-Marc, F-91460 Marcoussis (FR)
(74) Mandataire: Ilgart, Jean-Christophe
(86) Numéro de dépôt international: PCT/EP2010/054656
(87) Numéro de publication internationale: WO 2010/115963

(56) Documents cités:
- WO-A-2007/095971
- FR-A- 2 904 693
- US-A- 4 706 021
- US-A- 5 047 719
- US-A1- 2005 007 106
- US-A1- 2007 029 997

## Description

### DOMAINE TECHNIQUE

L'invention concerne un dispositif de contrôle non destructif d'une structure électriquement conductrice.

### ÉTAT DE LA TECHNIQUE ANTÉRIEURE

Le domaine de l'invention est celui du contrôle non destructif (CND) permettant la détection de défauts, telles que des entailles, fissures, corrosion,... dans des structures ou pièces conductrices.

Parmi toutes les méthodes utilisées, la méthode électromagnétique est particulièrement adaptée à l'inspection de structures conductrices de faible épaisseur pas forcément planes, telles que des structures aéronautiques en alliage léger ou des tubes de générateurs de vapeur dans les centrales nucléaires, dans lesquelles un manque de matière peut provoquer des fuites. Cette méthode consiste à émettre, à l'aide d'une partie inductrice, un champ électromagnétique au voisinage de la structure à inspecter et à mesurer, à l'aide d'une partie réceptrice (capteurs ou récepteurs de champ magnétique) la perturbation du flux magnétique engendrée par la présence d'un éventuel défaut dans la structure. L'ensemble comprenant la partie inductrice et la partie réceptrice de champ magnétique est appelée « sonde ».

Les récepteurs peuvent être soit de type inductif, soit de type magnétique par exemple AMR (Magnétorésistance anisotrope), GMR (Magnétorésistance géante), GMI (Magnéto-impédance géante), effet Hall.... Le domaine de l'invention concerne plus particulièrement la deuxième catégorie, c'est-à-dire les récepteurs de type magnétique.

Afin d'augmenter la rapidité des acquisitions, on peut utiliser des ensembles récepteur multiéléments, c'est-à-dire comportant de multiples récepteurs élémentaires identiques, dans une même sonde. Avec des barrettes 1D (ou une dimension), le déplacement de la sonde peut être réduit à un seul axe. Avec des imageurs magnétiques 2D (ou deux dimensions) un déplacement de la sonde n'est plus nécessaire. En pratique, il existe d'importants problèmes de connectique pour exploiter ces récepteurs magnétiques élémentaires, qui se présentent généralement sous la forme de quadripôles. Une place très importante dans la sonde doit donc être consacrée à la connectique, ce qui conduit à espacer les récepteurs élémentaires, et peut entraîner le masquage de la présence d'un défaut du fait de l'existence de zones non couvertes par un récepteur. De plus, une place importante est également prise par l'électronique, qui comprend souvent un amplificateur par récepteur élémentaire, et les moyens de multiplexage dans la sonde.

Le document référencé [1] en fin de description décrit l'utilisation de matrices ou barrettes de capteurs à courants de Foucault de type bobinage, avec une connexion série des bobinages émetteurs et des bobinages récepteurs. Les récepteurs de type magnétique (GMR,...) ne sont pas concernés.

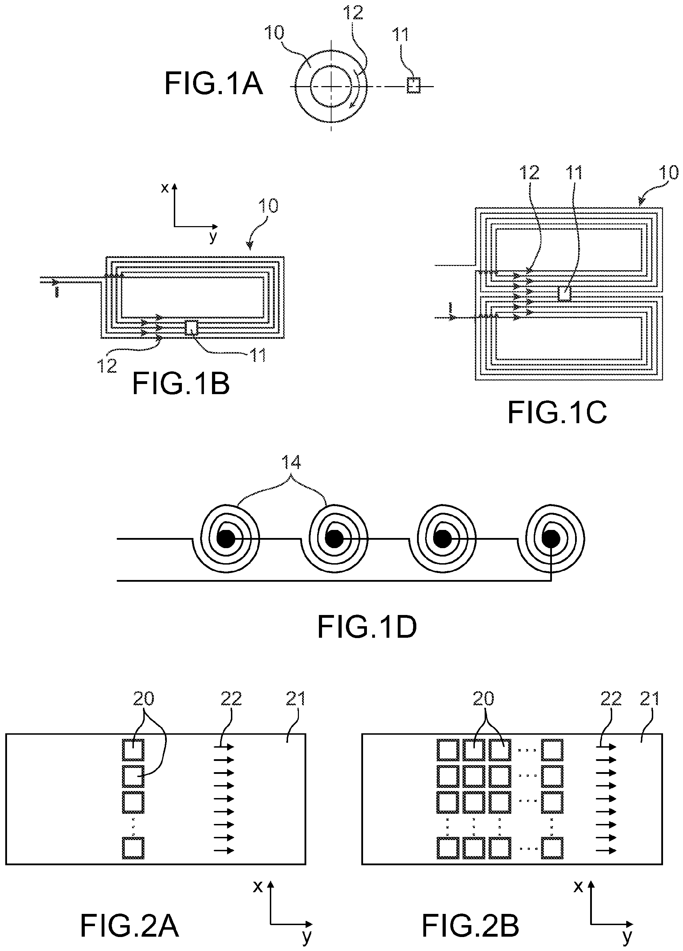

Les figures 1A à 1C présentent quelques dispositifs de l'art connu classiquement utilisés en contrôle non destructif avec un inducteur 10 et un récepteur magnétique 11 :
- L'inducteur 10 peut être un bobinage, comme illustré sur la figure 1A, le récepteur magnétique 11 étant situé à quelques millimètres ou dizaine de millimètres de ce bobinage, la flèche 12 indiquant le sens du courant.
- L'inducteur 10 peut être une "nappe" qui permet, sous les fils, de générer une distribution homogène locale du champ magnétique, comme illustré sur la figure 1B, le récepteur magnétique 11 étant disposé sous ces fils, les flèches 12 indiquant le sens du courant.
- L'inducteur 10 peut être une double nappe, comme illustré sur la figure 1C, le récepteur magnétique 11 étant disposé au centre de la nappe, les flèches 12 indiquant le sens du courant.

Le domaine de l'invention est plus particulièrement celui des inducteurs de type "nappe", tel qu'illustré sur les figures 1B ou 1C, et des récepteurs magnétiques de type GMR, ou également de type GMI, AMR, TMR (magnétorésistance tunnel), .... La détection magnétique peut être utilisée comme une fonction mélangeur, comme décrit dans le document [2]. On a alors une alimentation de la nappe à une fréquence f, cette fréquence étant généralement fixée en fonction du matériau étudié et des caractéristiques du défaut à détecter. L'alimentation, par exemple en courant, du détecteur magnétique peut être soit continue, soit alternative à une fréquence f'. Si l'alimentation est continue, la démodulation du signal complexe aux bornes du détecteur magnétique est réalisée à une fréquence f. Si elle est alternative, deux raies apparaissent dans le signal, l'une à la fréquence f-f' et l'autre à la fréquence f+f' et une démodulation à f-f' ou f+f' détermine l'amplitude de la tension complexe à cette fréquence. Après démodulation, l'amplitude est proportionnelle au courant qui traverse le détecteur et au champ magnétique dans lequel il baigne.

Dans le cas d'un ensemble récepteur multiéléments, comme illustré sur les figures 2A et 2B, les récepteurs élémentaires 20 sont classiquement disposés les uns à côté des autres et sont indépendants. L'inducteur nappe, représenté par un rectangle plein 21, recouvre l'ensemble de ces récepteurs élémentaires. Les flèches 22 indiquent le sens du courant. Dans le cas de la figure 2A, afin de couvrir une surface importante, l'ensemble récepteur est déplacé suivant l'axe Y. Dans le cas de la figure 2B, la sonde (inducteur + récepteurs) peut être utilisée sans mouvement et réaliser une cartographie statique du champ magnétique rayonné par les défauts dans une structure.

La connectique des dispositifs de l'art connu est complexe du fait de l'utilisation d'au moins une liaison par élément. Ainsi, une détection magnétique de type GMR nécessite classiquement deux fils d'alimentation et deux fils de mesure différentielle. Ceux-ci sont reliés à des amplificateurs différentiels. Puis des multiplexeurs/démultiplexeurs analogiques sont indispensables afin de réduire le nombre de liaisons entre la sonde et l'instrumentation. Celle-ci comprend, de manière classique, des démodulateurs synchrones pour exploiter les signaux en contrôle non destructif par courants de Foucault, et pour obtenir l'amplitude complexe de la raie à la fréquence de démodulation.

L'invention a pour objet de résoudre ces problèmes techniques en proposant un dispositif de contrôle comprenant un ensemble récepteur multiéléments sous la forme d'une barrette ou d'une matrice de récepteurs magnétiques élémentaires, qui permet de minimiser la connectique et l'électronique en alimentant les récepteurs magnétiques élémentaires en série ou en parallèle pour augmenter leur densité et minimiser les zones de la structure à contrôler non couvertes par un récepteur magnétique élémentaire.

### EXPOSÉ DE L'INVENTION

L'invention concerne un dispositif de contrôle non destructif d'une pièce électriquement conductrice tel que défini dans la revendication 1.

Chaque inducteur élémentaire peut être une nappe comprenant un fil conducteur, ou plusieurs brins conducteurs en série, ou plusieurs brins conducteurs en parallèle.

Dans un premier mode de réalisation, chaque colonne i de récepteurs est alimentée en courant, la tension mesurée aux bornes d'une colonne étant la somme d'au plus 2n tensions élémentaires sinusoïdales, aux fréquences f₁±f'i, f₂±f'ᵢ, ... et fₙ±f'ᵢ et des moyens de démodulation à f₁+f'ᵢ, f₂+f'ᵢ, ..., fₙ+f'ᵢ ou f₁-f'ᵢ, f₂-f'ᵢ, ..., fₙ-f'ᵢ.

Dans un second mode de réalisation, chaque colonne de récepteurs est alimentée en tension, le courant traversant une colonne i étant la somme d'au plus 2n courants élémentaires sinusoïdaux, aux fréquences f₁±f'i₁, f₂±f'ᵢ, ... et fₙ±f'ᵢ et des moyens de démodulation à f₁+f'ᵢ, f₂+f'ᵢ, ..., fₙ+f'ᵢ ou f₁-f'ᵢ, f₂-f'ᵢ, ..., fₙ-f'ᵢ.

Dans un troisième mode de réalisation, chaque colonne i de récepteurs constitue une branche d'un pont de wheatstone résistif, alimenté par une tension alternative et dont on mesure la tension différentielle de déséquilibre aux fréquences f₁±f'ᵢ. f₂±f'ᵢ, ..., fₙ±f'ᵢ et des moyens de démodulation à f₁+f'ᵢ, f₂+f'ᵢ, ..., fₙ+f'ᵢ ou f₁-f'ᵢ, f₂-f'ᵢ, ..., fₙ-f'ᵢ.

Avantageusement, les récepteurs magnétiques sont orientés selon l'axe X de sensibilité, la direction principale des courants de ces récepteurs élémentaires étant orientée selon l'axe Y.

Dans un exemple de réalisation, le dispositif de l'invention comprend une matrice de m x n récepteurs magnétiques (ie n'=n : chaque colonne i contient autant de récepteurs qu'il y a d'inducteurs élémentaires), les fréquences f₁, f₂, ..., fₙ sont respectivement égales à des fréquences f+Δf, f+2Δf, ..., f+nΔf et les fréquences f'₁, f'₂, ..., f'ₙ sont toutes égales à la fréquence f, la fréquence f peut être de l'ordre de 1 MHz et la fréquence Δf de l'ordre de 10 KHz.

Selon un autre exemple de réalisation, l'alimentation des récepteurs est réalisée en continu (f'₁=f'₂=...=f'ₙ=0) et les fréquences f₁, f₂, ..., fₙ sont respectivement égales à des fréquences f+Δf, f+2Δf, ..., f+nΔf. La fréquence f peut être de l'ordre de 1 MHz et la fréquence Δf de l'ordre de 20 KHz.

Avantageusement, le dispositif de l'invention comprend un support sur lequel sont disposés les inducteurs et les récepteurs magnétiques, qui peut être un circuit imprimé multicouches flexible. Ces récepteurs magnétiques peuvent être des récepteurs filaires continus. La partie induction peut comprendre plusieurs nappes réparties sur au moins une couche d'un substrat multicouches.

Avantageusement, les récepteurs magnétiques sont des récepteurs de type GMR.

Le dispositif de l'invention présente de nombreux avantages, et notamment :
- une minimisation de la connectique et une augmentation de la densité des récepteurs élémentaires,
- une minimisation de l'électronique de traitement au sein du dispositif,
- une simplification de la mise en oeuvre de l'ensemble récepteur magnétique,
- une possibilité de réaliser des imageurs 2D.

Le dispositif de l'invention peut être notamment utilisé pour des applications multiéléments de contrôle non destructif par courants de Foucault, et plus particulièrement pour des applications visant à détecter de petits défauts en surface d'une structure.

### BRÈVE DESCRIPTION DES DESSINS

Les figures 1A, 1B, 1C et 1D et 2A et 2B illustrent des dispositifs inducteur/récepteur de l'art connu.
La figure 3 illustre le dispositif de l'invention.
Les figures 4B à 4D illustrent l'allure de cartographies de champ magnétique obtenues suivant l'orientation du récepteur magnétique lors du déplacement d'un exemple de réalisation du dispositif de l'invention illustré sur la figure 4A.
La figure 5 illustre une exemple de réalisation du dispositif de l'invention.
Les figures 6 à 9B illustrent un autre exemple de réalisation du dispositif de l'invention, la figure 9B étant une vue en coupe de la figure 9A selon le plan A-A.
Les figures 10A et 10B illustrent des résultats expérimentaux obtenus avec l'exemple de réalisation du dispositif de l'invention tel qu'illustré sur les figures 9A et 9B.
La figure 11 et 12A à 12F illustrent d'autres exemples de réalisation du dispositif de l'invention.

### EXPOSÉ DÉTAILLÉ DE MODES DE RÉALISATION PARTICULIERS

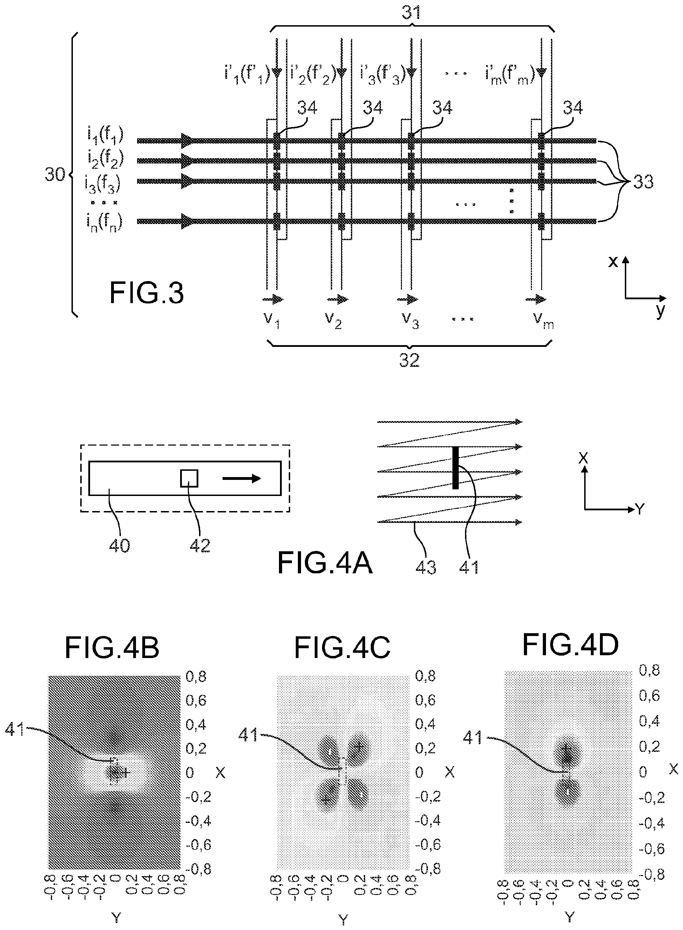

Le dispositif de l'invention, tel qu'illustré sur la figure 3, comprend :
- une partie inductrice comprenant un inducteur dissocié en n inducteurs élémentaires 33 alimentés à des fréquences f₁, f₂, ..., fₙ distinctes,
- une partie réceptrice comprenant n' récepteurs magnétiques 34 répartis sur m colonnes, chaque colonne comportant au plus n récepteurs magnétiques 34, ces récepteurs magnétiques étant reliés entre eux sur chaque colonne, les m colonnes de récepteurs étant alimentées par des signaux électriques v₁', v₂', ..., vₘ', de fréquences f₁', f₂', ..., fₘ', nulles ou non nulles, chaque récepteur magnétique 34 étant disposé sous un inducteur élémentaire, les indices n, n' et m étant des entiers positifs tels que n>=2, 1<n'<=n^{∗}m et m>=1
- des moyens de traitement permettant de connaître le champ magnétique en chacun des récepteurs magnétiques 34 d'une colonne.

On a une alimentation inducteur 30, une alimentation 31 des récepteurs magnétiques 34 et des voies de réception 32.

Les signaux électriques v₁', v₂', ..., vₘ' peuvent être soit des tensions ou courants continus, soit des tensions ou courants alternatifs de fréquences respectives f₁', f₂', ..., f_{m'}. Dans la suite du document, pour simplifier les cas, on considère le cas le plus général où les tensions v₁', v₂', ..., vₘ' sont soit sinusoïdales de fréquences f₁', f₂', ..., fₘ', soit continues en considérant dans ce cas f₁'=f₂'=...=fₘ'=0.

Chaque inducteur élémentaire 33 peut être une nappe comprenant un fil conducteur, ou plusieurs brins conducteurs en série comme illustré sur les figures 1B et 1C, ou plusieurs brins conducteurs en parallèle. Les brins peuvent ne pas être rectilignes. Ils peuvent être constitués par de petites nappes élémentaires alimentées par un même courant ou encore des bobinages 14, tel que représenté sur la figure 1D.

Chaque récepteur magnétique 34 est utilisé comme démodulateur. Ainsi, par exemple, la tension v₁ mesurée aux bornes de la première colonne de récepteurs magnétiques 34 mis en série est la somme de 2n tensions élémentaires sinusoïdales, à f₁±f'₁, f₂±f'₁, ..., et fₙ±f'₁. Des démodulations de v₁ à f₁-f'₁, f₂-f'₁, ..., et fₙ-f'₁ ou f₁+f'₁, f₂+f'₁, ..., et fₙ+f'₁ permettent d'en déduire l'amplitude complexe de la composante de v₁ à chacune de ces fréquences et par suite d'en déduire le champ magnétique présent en chacun des points de mesure, ou récepteurs, de la colonne. Des multiplexeurs ne sont pas nécessaires.

L'alimentation 31 des colonnes de récepteurs magnétiques 34 en série peut être une alimentation en tension, le courant traversant ces récepteurs étant alors le signal mesuré.

Dans un premier mode de réalisation, chaque colonne i comportant n'ᵢ récepteurs est alimentée en courant à la fréquence f'ᵢ, la tension mesurée aux bornes de cette colonne i étant la somme de 2n'ᵢ tensions élémentaires (n'ᵢ dans le cas où f'ᵢ=0), au plus aux fréquences f₁±f'ᵢ, f₂±f'ᵢ, ... et fₙ±f'ᵢ (l'amplitude à certaines de ces fréquences étant nulle ou très faible en l'absence d'un récepteur sous un inducteur élémentaire de la colonne).

Dans un deuxième mode de réalisation, chaque colonne i est alimentée en tension à la fréquence f'ᵢ, le courant traversant une colonne i étant la somme de 2n'ᵢ courants élémentaires, au plus aux fréquences f₁±f'ᵢ, f₂±f'ᵢ, ..., et fₙ±f'ᵢ.

Dans un troisième mode de réalisation, chaque colonne i comportant n'ᵢ récepteurs est insérée dans une branche d'un pont de Wheatstone. Les trois impédances du pont sont par exemple des résistances, chacune d'une valeur proche de la résistance de la colonne totale des récepteurs magnétiques. Chaque pont i est alimenté à la fréquence f'ᵢ. La tension différentielle de déséquilibre, mesurée aux bornes des branches intermédiaires du pont, est la somme de 2n'ᵢ tensions élémentaires, au plus aux fréquences f₁±f'ᵢ, f₂±f'ᵢ, ... et fₙ±f'ᵢ.

Dans tous ces cas, la démodulation est réalisée soit aux fréquences f₁-f'ᵢ, f₂-f'ᵢ, ... et fₙ-f'ᵢ soit aux fréquences f₁+f'ᵢ, f₂+f'ᵢ, ... et fₙ+f'ᵢ, afin de déterminer le champ magnétique local au niveau de chacun des récepteurs. Dans le cas particulier où l'alimentation de la colonne de récepteur est continue, la démodulation est nécessairement réalisée aux fréquences f₁, f₂, ... et fₙ puisque f₁'=f₂'=...=fₘ'=0.

On peut en choisissant bien les fréquences f₁, ..., fₙ, et f'₁, ..., f'ₘ, sommer les tensions de réception v₁, v₂,..., vₘ en un signal unique en utilisant des moyens d'amplification, et démoduler ce signal unique à l'aide de moyens de démodulation aux n x m différences de fréquences fᵢ-f'ⱼ. Un seul câble de réception est alors nécessaire entre le dispositif de l'invention (sonde) et les moyens de démodulation. Avec le dispositif de l'invention, le nombre de connexions est donc limité.

Les récepteurs magnétiques 34 possèdent un axe de sensibilité. Ils peuvent être préférentiellement orientés selon l'un des trois axes X, Y ou Z. Les figures 4B à 4D donnent l'allure des cartographies de champ magnétique obtenu lors du déplacement (balayage) 43 selon les axes X et Y d'un exemple de réalisation du dispositif de l'invention, illustré sur la figure 4A, qui comprend un inducteur élémentaire 40, orienté selon l'axe Y au-dessus d'une entaille 41 dans la structure à contrôler, et un récepteur magnétique 42. Ce récepteur magnétique 42 peut être orienté selon l'axe X, ce qui permet d'obtenir la cartographie illustrée sur la figure 4B, selon l'axe Y ce qui permet d'obtenir la cartographie illustrée sur la figure 4C, ou selon l'axe Z ce qui permet d'obtenir la cartographie illustrée sur la figure 4D. On obtient un signal globalement mono-polaire dans le cas illustré sur la figure 4B (axe X) en négligeant les petits lobes secondaires, de part et d'autre, d'amplitude nettement moins importante que le lobe central, un signal quadripolaire dans le cas illustré sur la figure 4C (axe Y) et un signal bipolaire dans le cas illustré sur la figure 4D (axe Z).

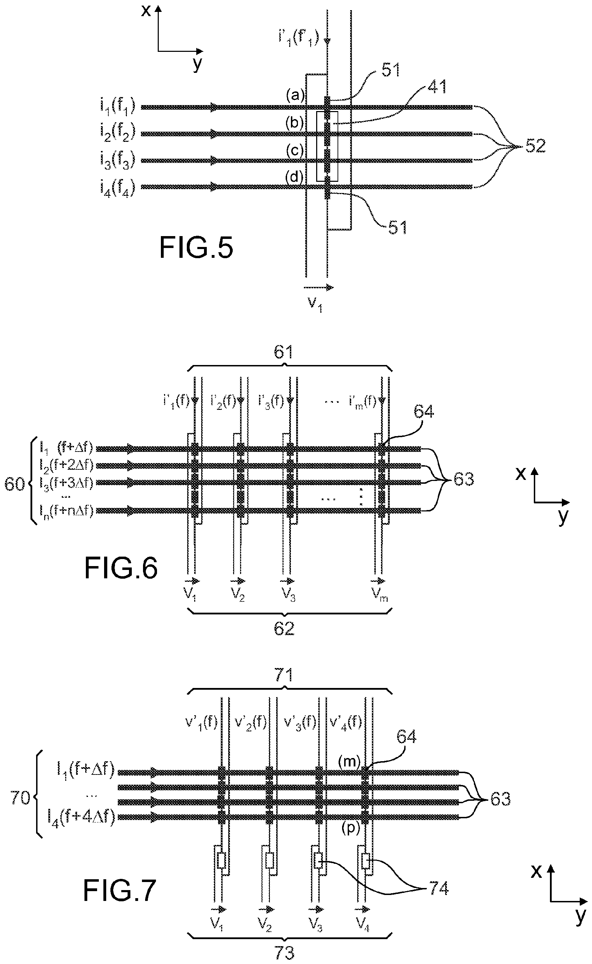

La figure 5 illustre un exemple de réalisation du dispositif de l'invention, dans lequel le défaut (entaille 41) est situé au centre d'un inducteur comprenant quatre inducteurs élémentaires 52, et de quatre récepteurs magnétiques correspondants 51, dénommés respectivement a, b, c et d. Le signal v₁ dû à l'entaille 41 résultant de la mise en série des quatre récepteurs magnétiques 51 est théoriquement nul dans le cas d'une orientation du récepteur selon l'axe Y ou l'axe Z : par raison de symétrie, les contributions des récepteurs magnétiques b et c sont identiques mais de signes opposés, de même pour les récepteurs magnétiques a et d. De telles configurations peuvent donc conduire à des non-détections de défauts, ce qui n'est pas le cas avec une orientation selon l'axe X du récepteur magnétique, du fait de la parité, selon les colonnes, de la cartographie illustrée sur la figure 4B. La configuration de mesure selon l'axe X est donc plus avantageuse dans le cadre du dispositif de l'invention.

Un exemple de réalisation simplifiée en termes de choix des fréquences est illustré sur la figure 6 avec une matrice de m x n récepteurs magnétiques, avec l'alimentation inducteur 60 des inducteurs élémentaires 63 à des fréquences f+Δf, f+2Δf, ..., f+nΔf, l'alimentation 61 des récepteurs 64 à la fréquence f et les voies de réception 62 permettant d'obtenir des sommes de signaux des fréquences Δf, 2Δf, 3Δf, ..., nΔf. Pour chacune des colonnes de récepteurs magnétiques 64, la démodulation est ainsi réalisée à Δf, 2Δf, ..., nΔf.

La figure 7 reprend l'exemple de réalisation simplifié de la figure 6 avec une matrice de 4 x 4 récepteurs magnétiques 64, tous orientés selon l'axe X. La fréquence de test f est de l'ordre du MHz et la fréquence Δf de l'ordre de 10 kHz. Les récepteurs magnétiques 64 utilisés sont plongés dans un champ magnétique continu de polarisation orienté selon leur axe de sensibilité afin qu'ils puissent fonctionner dans leur domaine de linéarité. Cette polarisation est réalisée par des fils conducteurs, alimentés avec une tension continue réglable, passant sous les récepteurs magnétiques. Sur la figure 7 sont également représentées l'alimentation inducteur 70 aux fréquences f+Δf, ..., f+4Δf, l'alimentation 71 des récepteurs magnétiques 64 à la fréquence f et les voies de réception 72 (somme des signaux aux fréquences Δf, 2Δf, 3Δf et 4Δf). La référence 74 représente des résistances de faible valeur de mesure de l'image du courant.

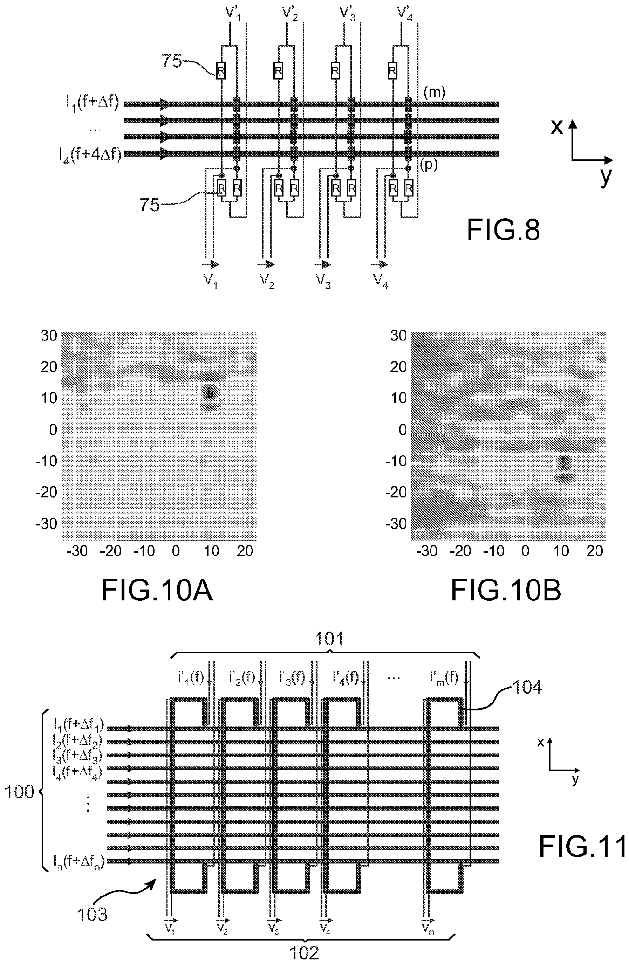

La figure 8 reprend l'exemple de la réalisation simplifiée de la figure 7 dans le troisième mode de réalisation, chaque colonne comportant 4 récepteurs étant insérée dans une branche d'un pont de Wheatstone (résistances 75).

Le support utilisé pour les récepteurs magnétiques 64 et les inducteurs élémentaires 63, qui peuvent être disposés de part et d'autre de celui-ci, est un circuit imprimé flexible, par exemple un circuit imprimé « flex » en époxy de 400 µm d'épaisseur. Un tel circuit permet au dispositif de l'invention d'épouser des formes complexes. Chacune des colonnes de récepteurs magnétiques est alimentée par une tension alternative v'₁, v'₂, v'₃ ou v'₄ et les résistances de faible valeur 74 insérées en série avec les récepteurs magnétiques 64 permettent chacune de mesurer une tension image du courant dans les récepteurs magnétiques 64. Cette tension comprend notamment des raies aux fréquences Δf, 2Δf, 3Δf et 4Δf, qui, après démodulation à ces fréquences, permettent de détecter la présence éventuelle d'une entaille dans la structure ou à la pièce à contrôler.

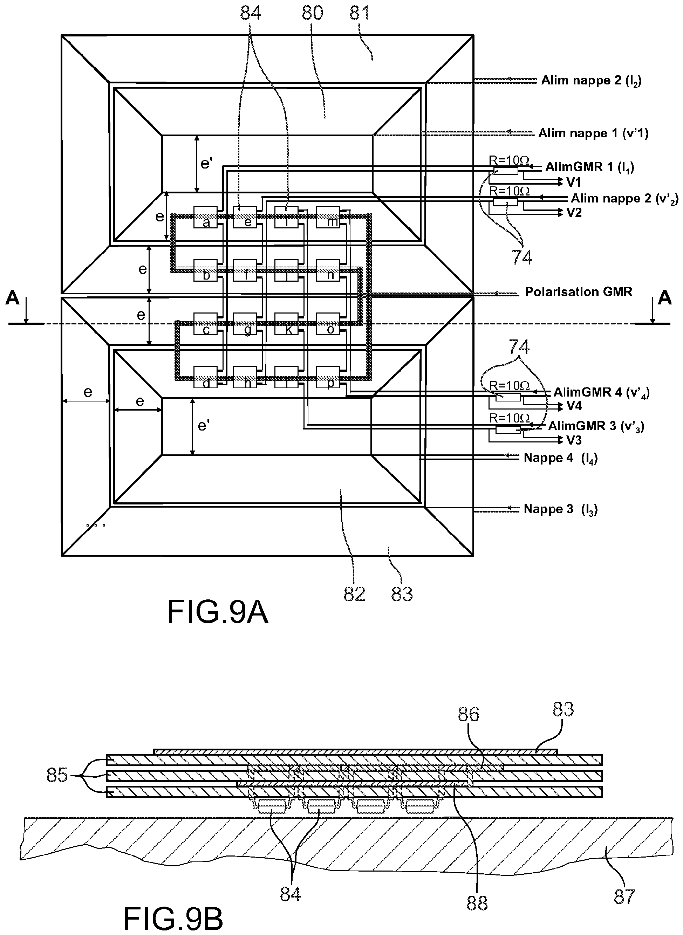

Les figures 9A et 9B représentent l'exemple de réalisation de la figure 7, en illustrant quatre inducteurs élémentaires 80, 81, 82 et 83, et 4 x 4 récepteurs magnétiques 84, par exemple de type GMR, référencés respectivement par les lettres a à p, avec leurs alimentations respectives. Les récepteurs a, e, i, m sont ainsi disposés sous la nappe 80, les récepteurs b, f, j, n sous l'inducteur élémentaire 81, les récepteurs c, g, k, o sous l'inducteur élémentaire 82 et les récepteurs d, h, l, p sous l'inducteur élémentaire 84. Les largeurs e des inducteurs élémentaires représentés peuvent être, par exemple, égales à 7 mm et la largeur e' de l'ouverture interne des inducteurs élémentaires 80 et 82 égale à 8 mm.

Comme illustré sur la figure 9B, les inducteurs élémentaires 80, 81, 82 et 83, d'une part, et les récepteurs magnétiques 84 d'autre part, sont disposés de part et d'autre d'un support 85, constitué ici d'un circuit imprimé quatre couches. Le connectique 86, et la bobine de polarisation 88 étant disposées entre ces couches. La référence 87 illustre la pièce à contrôler.

Le dispositif de l'invention ainsi illustré sur les figures 9A et 9B peut être testé par balayage au-dessus d'une pièce plane comprenant un défaut électro-érodé de 10 mm de longueur réalisé en surface. Les figures 10A et 10B illustrent le signal obtenu, à l'aide des récepteurs magnétiques référencés n et p, après démodulation de la tension v₄ aux fréquences Δf et 4Δf. Les deux récepteurs magnétiques m et p permettent de bien détecter ce défaut de manière totalement indépendante et sans diaphonie.

On peut réaliser un ensemble récepteur magnétique multiéléments avec des récepteurs magnétiques en forme de « C » ("yoke") 103, comme illustré sur la figure 11, dont l'espace entre deux élément récepteur magnétique 104 voisins, est de 100 µm environ. Les nécessités de connexion empêchent la réalisation d'une matrice 2D compte-tenu de l'encombrement nécessaire pour les contacts d'interconnexion. Mais on peut ne conserver que deux contacts extérieurs pour chaque récepteur élémentaire, comme illustré sur la figure 11, en utilisant, comme précédemment, un circuit pour démoduler et exploiter les signaux issus des différents éléments récepteurs magnétiques. Sur la figure 11 sont représentées l'alimentation inducteur 100, l'alimentation récepteurs 101, et les voies réception 102 (somme des tensions à Δf₁, Δf₂, ..., Δfₙ). Ainsi, les récepteurs magnétiques élémentaires 104 ne sont plus discrets mais continus. Un tel ensemble récepteur présente d'autres avantages, et notamment :
- On peut utiliser des sondes à courants de Foucault avec une grande densité de capteurs.
- Les capacités parasites ramenées via les contacts sont supprimées, ce qui est particulièrement avantageux à des fréquences dépassant quelques centaines de kHz.
- La réalisation et la connexion de l'ensemble récepteur magnétique se trouvent largement simplifiées.
- On peut minimiser l'électronique d'amplification faible bruit proche capteur si nécessaire, en utilisant un amplificateur par colonne au lieu de un par élément.
- L'ensemble récepteur magnétique peut être très long, par exemple plusieurs centimètres.

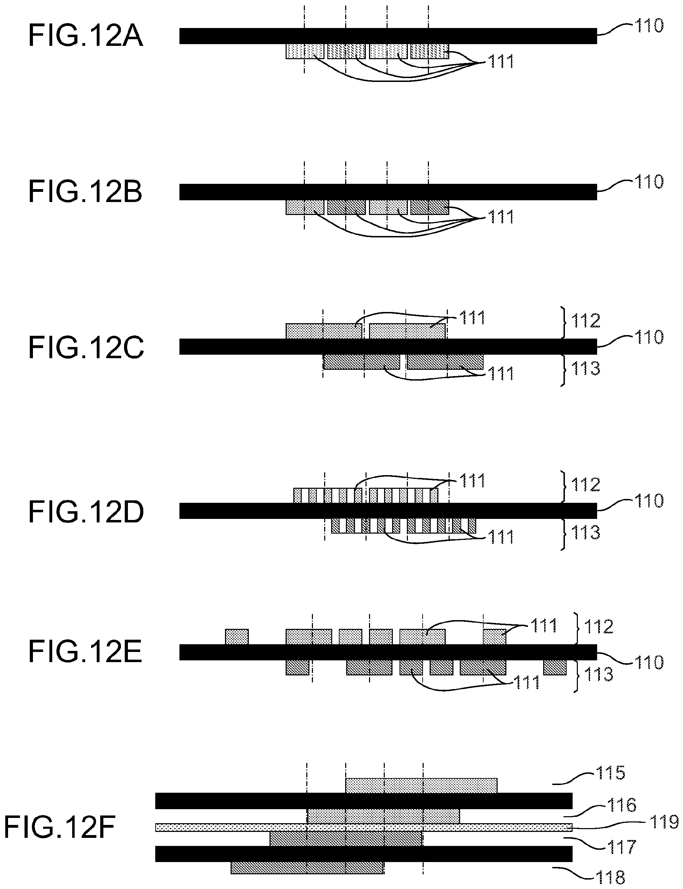

Dans le dispositif de l'invention, les inducteurs élémentaires 111 constituant la partie inductrice peuvent être réalisés soit avec un seul conducteur massif (fil), soit avec différents brins conducteurs mis en série ou en parallèle. Comme illustré sur les figures 12A à 12F, les brins d'un même inducteur élémentaire 111 peuvent être éventuellement réparties sur différentes couches d'un substrat multicouche 110 (circuit imprimé, kapton,...). Les brins successifs peuvent également être partiellement superposés en utilisant un substrat multicouche, ou encore imbriqués. Les figures 12A à 12F illustrent différents exemples de réalisation pour quatre inducteurs élémentaires 111 disposées sur une couche comme illustré sur les figures 12A et 12B, sur deux couches 112 et 113 comme illustré sur les figures 12C à 12E ou sur quatre couches 115, 116, 117, 118 comme illustré sur la figure 12F, la couche 119 étant une couche d'isolant.

Dans des variantes de réalisation du dispositif de l'invention, on peut utiliser :
- des récepteurs magnétiques en parallèle au lieu d'être mise en série,
- des inducteurs élémentaires sous forme de grands bobinages circulaires ou de mises en série de petits bobinages,
- un ruban magnétique au-dessus des inducteurs élémentaires pour augmenter le champ inducteur,
- une disposition des récepteurs magnétiques en quinconce,
- un substrat souple (kapton ou circuit imprimé flexible),
- l'utilisation de récepteurs magnétiques de type GMR ou de type quadripôle : AMR, TMR, GMI,...,
- un fonctionnement avec des récepteurs magnétiques intégrés montés en pont,
- l'utilisation de récepteurs avec des éléments magnétiques pour concentrer le flux dans la réception magnétique selon l'axe de sensibilité.

### REFERENCES

[1] FR 2 904 693
[2] WO 2007/095971

## Revendications

1. Dispositif de contrôle non destructif d'une pièce électriquement conductrice comprenant :
- une partie inductrice, et
- une partie réceptrice,
- des moyens de traitement,
**caractérisé en ce que** la partie inductrice comprend un inducteur dissocié en n inducteurs élémentaires de type nappe (33) alimentés à des fréquences f₁, f₂, ..., fₙ distinctes,
**en ce que** la partie réceptrice comprend n' récepteurs magnétiques (34) répartis sur m colonnes, chaque colonne comportant au plus n récepteurs, au moins une colonne comportant au moins deux récepteurs, les récepteurs magnétiques étant reliés entre eux sur chaque colonne, les m colonnes de récepteurs étant alimentées par des signaux électriques v₁', v₂', ..., vₘ', de fréquences f₁', f₂', ..., fₘ', nulles ou non nulles, chaque récepteur magnétique (34) étant disposé sous un inducteur élémentaire, m récepteurs magnétiques étant disposés sous chaque inducteur élémentaire, les indices n, n' et m étant des entiers positifs tels que n>=2, 1<n'<=n^{∗}m et m>=1, **en ce que** les moyens de traitement comprennent, pour chaque colonne de récepteurs de rang i avec 1 ≤ i ≤ m, des moyens de mesure de signal aux bornes de cette colonne de récepteurs suivis de moyens de démodulation de ce signal aux fréquences f₁ + fᵢ', f₂ + fᵢ' --- fₙ + fᵢ' ou f₁ - fᵢ', f₂ - fᵢ' --- fₙ - fᵢ' et de moyens de calcul du champ magnétique en chacun des récepteurs de cette colonne, et **en ce que** les récepteurs magnétiques (34) sont reliés en série ou en parallèle.

2. Dispositif selon la revendication 1, dans lequel chaque inducteur élémentaire (33) est une nappe comprenant un fil conducteur, ou plusieurs brins conducteurs en série, ou plusieurs brins conducteurs en parallèle.

3. Dispositif selon la revendication 1, dans lequel chaque colonne i de récepteurs est alimentée en courant, la tension mesurée aux bornes d'une colonne étant la somme d'au plus 2n tensions élémentaires sinusoïdales, aux fréquences f₁±f'ᵢ, f₂±f'ᵢ, ... et fₙ+f'ᵢ et des moyens de démodulation à f₁+f'ᵢ, f₂+f'ᵢ, ..., fₙ+f'ᵢ ou f₁-f'ᵢ, f₂-f'ᵢ, ..., fₙ-f'ᵢ.

4. Dispositif selon la revendication 1, dans lequel chaque colonne i de récepteurs est alimentée en tension, le courant traversant une colonne étant la somme d'au plus 2n courants élémentaires sinusoïdaux, aux fréquences f₁±f'ᵢ, f₂±f'ᵢ, ... et fₙ±f'ᵢ et des moyens de démodulation à f₁+f'ᵢ, f₂+f'ᵢ, ..., fₙ+f'ᵢ ou f₁-f'ᵢ, f₂-f'ᵢ, ..., fₙ-f'ᵢ.

5. Dispositif selon la revendication 1, dans lequel chaque colonne i de récepteurs constitue une branche d'un pont de wheatstone résistif, alimenté par un signal à la fréquence f'ᵢ et dont on mesure la tension différentielle de déséquilibre aux fréquences f₁±f'ᵢ, f₂±f'ᵢ, ..., fₙ±f'ᵢ et des moyens de démodulation à f₁+f'ᵢ, f₂+f'ᵢ, ..., fₙ+f'ᵢ ou f₁-f'ᵢ, f₂-f'ᵢ, ..., fₙ-f'ᵢ.

6. Dispositif selon la revendication 1, comprenant une matrice de m x n récepteurs magnétiques (64) et dans lequel les fréquences f₁, f₂, ..., fₙ sont respectivement égales à des fréquences f+Δf, f+2Δf, ..., f+nΔf et dans lequel les fréquences f'₁, f'₂, ..., f'ₘ sont toutes égales à la fréquence f.

7. Dispositif selon la revendication 1, comprenant une matrice de m x n récepteurs magnétiques (64) et dans lequel les fréquences f₁, f₂, ..., fₙ sont respectivement égales à des fréquences f+Δf, f+2Δf, ..., f+nΔf et dans lequel les signaux v'₁, v'₂, ..., v'ₘ sont continus.

8. Dispositif selon la revendication 6, dans lequel la fréquence f est de l'ordre de 1 MHz et la fréquence ΔF de l'ordre de 10 KHz.

9. Dispositif selon la revendication 1, dans lequel les inducteurs élémentaires (33) et les récepteurs magnétiques (34) sont disposés sur un support.

10. Dispositif selon la revendication 9, dans lequel le support est un circuit imprimé flexible.

11. Dispositif selon la revendication 9, dans lequel le support est un circuit imprimé multicouches.

12. Dispositif selon la revendication 1 comprenant des récepteurs magnétiques filaires continus.

13. Dispositif selon la revendication 1, dans lequel la partie induction comprend plusieurs nappes réparties sur au moins une couche d'un substrat multicouche.

14. Dispositif selon la revendication 1, dans lequel les récepteurs magnétiques sont des récepteurs de type GMR.

15. Dispositif selon la revendication 1, dans lequel l'inducteur est couplé à un ruban magnétique.

## Patentansprüche

1. Vorrichtung zur zerstörungsfreien Kontrolle eines elektrisch leitenden Bauteils, umfassend Folgendes:
- ein Induktionsteil, und
- ein Empfangsteil,
- Mittel zur Verarbeitung,
**dadurch gekennzeichnet, dass** das Induktionsteil einen Induktor umfasst, der in n Elementarinduktoren vom Typ Lage (33) dissoziiert ist, die mit verschiedenen Frequenzen f₁, f₂, ..., fₙ versorgt werden,
dass das Empfangsteil n' magnetische Empfänger (34) umfasst, die auf m Spalten verteilt sind, wobei jede Spalte höchstens n Empfänger umfasst, wobei wenigstens eine Spalte wenigstens zwei Empfänger umfasst, wobei die magnetischen Empfänger miteinander in jeder Spalte verbunden sind, wobei die m Spalten von Empfängern durch elektrische Signale v₁', v₂', ..., vₘ' mit Frequenzen f₁', f₂', ..., fₘ' versorgt werden, die Null oder nicht Null sind, wobei jeder magnetische Empfänger (34) unter einem Elementarinduktor angeordnet ist, wobei m magnetische Empfänger unter jeden Elementarinduktor angeordnet sind, wobei die Indizes n, n' und m positive ganze Zahlen derart sind, dass n >= 2, 1 < n'< n^{∗}m und m >= 1, dass die Verarbeitungsmittel für jede Spalte von Empfängern mit Rang i mit 1 ≤ i ≤ m Mittel zum Messen eines Signals an den Anschlüssen dieser Spalte von Empfängern umfassen, gefolgt von Mitteln zum Demodulieren dieses Signals bei den Frequenzen f₁ + fᵢ', f₂ + fᵢ' --- fₙ + fᵢ' oder f₁ - fᵢ', f₂ - fᵢ' --- fₙ - fᵢ', und von Mitteln zum Berechnen des magnetischen Felds an jedem der Empfänger dieser Spalte, und dass die magnetischen Empfänger (34) in Reihe oder parallel verbunden sind.

2. Vorrichtung nach Anspruch 1, wobei jeder Elementarinduktor (33) eine Lage ist, umfassend einen leitenden Draht, oder mehrere leitende Adern in Reihe oder mehrere leitende Adern parallel.

3. Vorrichtung nach Anspruch 1, wobei jede Spalte i von Empfängern mit Strom versorgt wird, wobei die an den Anschlüssen einer Spalte gemessene Spannung die Summe ist von höchstens 2n sinusförmigen Elementarspannungen mit Frequenzen f₁ ± f'ᵢ, f₂ ± f'ᵢ, ... und fₙ ± f'ᵢ und der Demodulationsmittel mit f₁ + f'ᵢ, f₂ + f'ᵢ, ..., fₙ + f' oder f₁ - f'ᵢ, f₂ - f'ᵢ, ..., fₙ - f'ᵢ.

4. Vorrichtung nach Anspruch 1, wobei jede Spalte i von Empfängern mit Spannung versorgt wird, wobei der Strom, der eine Spalte durchfließt, die Summe ist von höchstens 2n sinusförmigen Elementarströmen mit Frequenzen f₁ ± f'ᵢ, f₂ ± f'ᵢ, ... und fₙ ± f'ᵢ und der Demodulationsmittel mit f₁ + f'ᵢ, f₂ + f'ᵢ, ..., fₙ + f' oder f₁ - f'ᵢ, f₂ - f'ᵢ, ..., fₙ - f'ᵢ.

5. Vorrichtung nach Anspruch 1, wobei jede Spalte i von Empfängern einen Zweig einer Widerstands-Wheatstonebrücke bildet, die mit einem Signal mit der Frequenz f'ᵢ versorgt wird und von der man die differenzielle Ungleichgewichtsspannung misst mit Frequenzen f₁ ± f'ᵢ, f₂ ± f'ᵢ, ..., fₙ ± f'ᵢ und der Demodulationsmittel mit f₁ + f'ᵢ, f₂ + f'ᵢ, ..., fₙ + f' oder f₁ - f'ᵢ, f₂ - f'ᵢ, ..., fₙ - f'ᵢ.

6. Vorrichtung nach Anspruch 1, umfassend eine Matrix von m x n magnetischen Empfängern (64), und wobei die Frequenzen f₁, f₂, ..., fₙ gleich jeweiligen Frequenzen f + Δf, f + 2Δf, ..., f + nΔf sind, und wobei die Frequenzen f'₁, f'₂, ..., f'ₘ alle gleich der Frequenz f sind.

7. Vorrichtung nach Anspruch 1, umfassend eine Matrix von m x n magnetischen Empfängern (64), und wobei die Frequenzen f₁, f₂, ..., fₙ gleich jeweiligen Frequenzen f + Δf, f + 2Δf, ..., f + nΔf sind, und wobei die Signale v'₁, v'₂, ..., v'ₘ kontinuierlich sind.

8. Vorrichtung nach Anspruch 6, wobei die Frequenz f in der Größenordnung von 1 MHz ist, und die Frequenz ΔF in der Größenordnung von 10 KHz ist.

9. Vorrichtung nach Anspruch 1, wobei die Elementarinduktoren (33) und die magnetischen Empfänger (34) auf einem Träger angeordnet sind.

10. Vorrichtung nach Anspruch 9, wobei der Träger eine flexible gedruckte Schaltung ist.

11. Vorrichtung nach Anspruch 9, wobei der Träger eine gedruckte Mehrschichtschaltung ist.

12. Vorrichtung nach Anspruch 1, umfassend kontinuierliche drahtförmige magnetische Empfänger.

13. Vorrichtung nach Anspruch 1, wobei das Induktionsteil mehrere Lagen umfasst, die auf wenigstens einer Schicht eines Mehrschichtsubstrats verteilt sind.

14. Vorrichtung nach Anspruch 1, wobei die magnetischen Empfänger Empfänger vom Typ GMR sind.

15. Vorrichtung nach Anspruch 1, wobei der Induktor mit einem magnetischen Band gekoppelt ist.

## Claims

1. Device for the non-destructive testing of an electrically conductive part comprising:
- an induction portion, and
- a receiving portion,
- processing means,
**characterised in that** the induction portion comprises an inductor dissociated into n elementary inductors of the web type (33) supplied at different frequencies f1, f2, ..., fn,
**in that** the receiving portion comprises n' magnetic receivers (34) distributed over m columns, each column comprising at most n receivers, at least one column comprising at least two receivers, the magnetic receivers being connected to one another over each column, the m columns of receivers being supplied by electrical signals v1', v2', ..., vm', of frequencies f1', f2', ..., fm', zero or non-zero, each magnetic receiver (34) being arranged under an elementary inductor, m magnetic receivers being positioned under each elementary inductor, the indices n, n' and m being positive integers such that n>=2, 1<n' <=n^{∗}m and m>=1, **in that** the processing means comprise, for each column of receivers of rank i, with 1 ≤ i ≤ m, means for measuring a signal at the terminals of this column of receivers followed by means of demodulating this signal at frequencies f₁+fᵢ', f₂+fᵢ', ..., fₙ+fᵢ' or f₁-fᵢ', f₂-fᵢ', ..., fₙ-fᵢ' and means of calculating a magnetic field at each of the receivers of this column, and **in that** the magnetic receivers (34) are connected in series or in parallel.

2. Device according to claim 1, wherein each elementary inductor (33) is a web comprising a conductive wire, or several conductive strands in series, or several conductive strands in parallel.

3. Device according to claim 1, wherein each column i of receivers is supplied in current, the voltage measured at the terminals of a column being the sum of at most 2n sinusoidal elementary voltages, at the frequencies f₁±f'ᵢ, f₂±f'ᵢ, ... and fₙ±f'ᵢ and means of demodulating at f₁+f'ᵢ, f₂+f'ᵢ, ..., fₙ+f'ᵢ or f₁-f'ᵢ, f₂-f'ᵢ, ..., fₙ-f'ᵢ.

4. Device according to claim 1, wherein each column i of receivers is supplied in voltage, the current passing through a column being the sum of at most 2n sinusoidal elementary currents, at the frequencies f₁±f'ᵢ, f₂±f'ᵢ, ... and fₙ±f'ᵢ and of means of demodulating at f₁+f'ᵢ, f₂+f'ᵢ, ..., fₙ+f'ᵢ or f₁-f'ᵢ, f₂-f'ᵢ, ..., fₙ-f'ᵢ.

5. Device according to claim 1, wherein each column i of receivers constitutes a branch of a Wheatstone resistive bridge, supplied by a signal at the frequency f'ᵢ and of which the differential imbalance voltage is measured at the frequencies f₁±f'ᵢ, f₂±f'ᵢ, ..., fₙ±f'ᵢ and of means of demodulating at f₁+f'ᵢ, f₂+f'ᵢ, ..., fₙ+f'ᵢ or f₁-f'ᵢ, f₂-f'ᵢ, ..., fₙ-f'ᵢ.

6. Device according to claim 1, comprising a matrix of m x n magnetic receivers (64) and wherein the frequencies f₁, f₂, ..., fₙ are respectively equal to frequencies f+Δf, f+2Δf, ..., f+nΔf and wherein the frequencies f'₁, f'₂, ..., f'ₘ are all equal to the frequency f.

7. Device according to claim 1, comprising a matrix of m x n magnetic receivers (64) and wherein the frequencies f₁, f₂, ..., fₙ are respectively equal to frequencies f+Δf, f+2Af, ..., f+nΔf and wherein the signals v'₁, v'₂, ..., v'ₘ are direct.

8. Device according to claim 6, wherein the frequency f is about 1 MHz and the frequency ΔF about 10 KHz.

9. Device according to claim 1, wherein the elementary inductors (33) and the magnetic receivers (34) are positioned on a support.

10. Device according to claim 9, wherein the support is a flexible printed circuit.

11. Device according to claim 11, wherein the support is a multi-layer printed circuit.

12. Device according to claim 1 comprising direct wired magnetic receivers.

13. Device according to claim 1, wherein the induction portion comprises several webs distributed over at least one layer of a multi-layer substrate.

14. Device according to claim 1, wherein the magnetic receivers are receivers of the GMR type.

15. Device according to claim 1, wherein the inductor is coupled to a magnetic tape.
